# EUROPEAN PATENT SPECIFICATION

(11) **EP 4 453 865 B1**
(45) Date of publication and mention of the grant of the patent: **19.08.2026**
(21) Application number: 22830878.9
(22) Date of filing: 20.12.2022
(51) Int. Cl.: G06T 7/11, A61B 5/06, G06T 7/73, G16H 30/40

(54) **SYSTEM FOR CREATING COMPOSITE CAMERA IMAGES FOR BODY SURFACE AREA MODELING AND DE-IDENTIFICATION OF PATIENTS IN ULTRASOUND IMAGING EXAMS**
SYSTEM ZUR ERZEUGUNG VON ZUSAMMENGESETZTEN KAMERABILDERN ZUR KÖRPEROBERFLÄCHENBEREICHSMODELLIERUNG UND -DEIDENTIFIZIERUNG VON PATIENTEN BEI ULTRASCHALLBILDGEBUNGSUNTERSUCHUNGEN
SYSTÈME DE CRÉATION D'IMAGES DE CAMÉRA COMPOSITE POUR MODÉLISATION DE ZONE DE SURFACE CORPORELLE ET DÉ-IDENTIFICATION DE PATIENTS DANS DES EXAMENS D'IMAGERIE ULTRASONORE

(30) Priority: 21.12.2021 US 202163292149 P
(43) Date of publication of application: 30.10.2024
(73) Proprietor: Koninklijke Philips N.V., 5656 AE Eindhoven (NL)
(72) Inventor: ERRICO, Claudia, 5656 AG Eindhoven (NL); BHARAT, Shyam, 5656 AG Eindhoven (NL); MANKOVICH, Gabriel Ryan, 5656 AG Eindhoven (NL); STAROBINETS, Olga, 5656 AG Eindhoven (NL); PREVRHAL, Sven Peter, 5656AG Eindhoven (NL)
(74) Representative: Philips Intellectual Property & Standards
(86) International application number: PCT/EP2022/086822
(87) International publication number: WO 2023/118026

(56) References cited:
- EP-A1- 3 188 058
- JP-A- 2021 069 698
- US-A1- 2020 118 317
- US-A1- 2021 209 734
- XU WANXIN ET AL: "Human body reshaping and its application using multiple RGB-D sensors", SIGNAL PROCESSING. IMAGE COMMUNICATION, ELSEVIER SCIENCE PUBLISHERS, AMSTERDAM, NL, vol. 79, 15 September 2019 (2019-09-15), pages 71 - 81, XP085866090, ISSN: 0923-5965, [retrieved on 20190915], DOI: 10.1016/J.IMAGE.2019.08.011

## Description

### BACKGROUND

Cameras are used in different settings to monitor workflow. Whether it is a medical application or not, the usage of cameras to improve operational efficiency has become widespread in the last decade.

In medical imaging, the usage of cameras has increased in recent years mainly to track equipment and medical personnel, and/or to predict and avoid workflow bottlenecks in hospital departments and patient care. While in CT and MR the patient can be imaged fully clothed, in ultrasound imaging the probe must touch the skin surface of the patient. Therefore, the areas of interest must be uncovered. The utilization of cameras then becomes challenging as we aim to preserve patients' privacy.

What is needed, therefore, is a method and system providing workflow imaging of a patient that overcomes at least the drawbacks of known methods and systems described above.

JP 2021 069698 A discloses a medical image capture procedure together with a camera where images are masked for privacy.

### SUMMARY

The invention is defined by the independent claims. Advantageous embodiments are provided in the dependent claims.

According to an aspect of the present disclosure, a method of performing ultrasound imaging is disclosed. The method comprises: receiving images from a camera; reconstructing a body surface map from the images from the camera; applying a first trained computational model to the body surface map to predict a body type; applying a second trained computational model to predict a pose of the body; and identifying, on the body surface map, a portion of the body to be obscured, and a portion of the body to be imaged during the ultrasound imaging method.

According to another aspect of the present disclosure, a system for medical imaging, comprises: a camera adapted to capture an image; a memory adapted to store a first computational model comprising first instructions, and a second computational model comprising second instructions; and a processor. The first instructions or the second instructions, when executed by the processor, cause the processor to: receive images from the camera; reconstruct a body surface map from the images from the camera; to predict a body type based on the body surface map; predict a pose of the body; and identify on the body surface map, a portion of the body to be obscured, and a portion of the body to be imaged during the medical imaging.

According to another aspect of the present disclosure, tangible, non-transitory computer readable medium stores a first computational model comprising first instructions and a second computational model comprising second instructions. The first instructions or the second instructions, when executed by a processor, cause the processor to: receive images from a camera; reconstruct a body surface map from the images from the camera; predict a body type based on the body surface map; predict a pose of the body; and identify on the body surface map, a portion of the body to be obscured, and a portion of the body to be imaged.

### BRIEF DESCRIPTION OF THE DRAWINGS

The example embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.
Fig. 1 is a simplified block diagram of a system for imaging a portion of a body, according to a representative embodiment.
Fig. 2 is a simplified flow diagram of a method to capture body surface maps and predict a body type in accordance with a representative embodiment.
Fig. 3 is a simplified flow diagram of a method to predict body pose, and probe location and orientation in accordance with a representative embodiment.
Figs. 4A-4E is a flow diagram of an ultrasound imaging procedure using computational models according to a representative embodiment.
Figs. 5 shows the orientation and movement of an ultrasound imaging probe according to a representative embodiment.
Fig. 6 is a simplified flow diagram showing a method of performing ultrasound imaging using a trained computational model, according to a representative embodiment.

### DETAILED DESCRIPTION

In the following detailed description, for the purposes of explanation and not limitation, representative embodiments disclosing specific details are set forth in order to provide a thorough understanding of an embodiment according to the present teachings. Descriptions of known systems, devices, materials, methods of operation and methods of manufacture may be omitted so as to avoid obscuring the description of the representative embodiments. Nonetheless, systems, devices and methods that are within the purview of one of ordinary skill in the art are within the scope of the present teachings and may be used in accordance with the representative embodiments. It is to be understood that the terminology used herein is for purposes of describing particular embodiments only and is not intended to be limiting. The defined terms are in addition to the technical and scientific meanings of the defined terms as commonly understood and accepted in the technical field of the present teachings.

It will be understood that, although the terms first, second, third, etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

The terminology used herein is for purposes of describing particular embodiments only and is not intended to be limiting. As used in the specification and appended claims, the singular forms of terms "a," "an" and "the" are intended to include both singular and plural forms, unless the context clearly dictates otherwise. Additionally, the terms "comprises," "comprising," and/or similar terms specify the presence of stated features, elements, and/or components, but do not preclude the presence or addition of one or more other features, elements, components, and/or groups thereof. As used herein, the term "and/or" includes any and all combinations of one or more of the associated listed items.

Unless otherwise noted, when an element or component is said to be "connected to," "coupled to," or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

The present disclosure, through one or more of its various aspects, embodiments and/or specific features or sub-components, is thus intended to bring out one or more of the advantages as specifically noted below. For purposes of explanation and not limitation, example embodiments disclosing specific details are set forth in order to provide a thorough understanding of an embodiment according to the present teachings. However, other embodiments consistent with the present disclosure that depart from specific details disclosed herein remain within the scope of the appended claims. Moreover, descriptions of well-known apparatuses and methods may be omitted so as to not obscure the description of the example embodiments. Such methods and apparatuses are within the scope of the present disclosure.

By the present teachings, among other things, images from cameras are used to create a digital contour map of the patient's body surface area, and to mask and blur patient's identity and body parts that are excluded from the imaging protocols. Isolation of body parts will follow the ultrasound (US) imaging protocol as well as the modelled patient's body surface area. Eventually, the camera will be also focusing on the probe motion on the patient's body surface (i.e. tracking the probe on the body surface) and will store that information along with the Digital Imaging and Communications in Medicine (DICOM) feed and the patient position during the imaging exam. The disadvantage of increased risk of privacy loss as a result of camera recording is overcome by image processing of the camera recording that converts exposed patient body surface to triangulated digital surface maps that no longer include privacy-sensitive visual detail. In some clinical applications, however, due to sensitive body parts being scanned, the user can disable acquisition and the imaging workflow will continue without camera-guided assistance.

The utilization of cameras in ultrasound imaging could help with workflow and operational efficiency and eventually lead to higher quality exams. However, ultrasound requires body parts to be uncovered during the imaging exam and this could become an obstacle unless de-identification, blurring and other masking techniques are applied to preserve patients' privacy. As will become clearer as the present description continues, ceiling/wall-mounted cameras are used to retrieve a map of the patients' body surface area. Once acquired, the maps are saved and processed to help identify patients' body parts, and to model body habitus and shape before the ultrasound imaging exam starts. Areas to be studied during the imaging exam protocol are isolated, and areas that are outside the imaging scanning protocols scans are obscured to avoid compromising patient anonymity. Notably, the region being imaged may also be obscured so as to not be seen by those beside, such as the clinician performing the imaging procedure. This obscuring is contemplated, for example, when the area to be studied is in a sensitive or private region of the body, such as the face, genitals, and/or breasts, for example. Alternatively, for sensitive areas, the body parts part of the scanning protocol might be represented with a mesh of the body surface area, while the rest of the body parts are either blurred or/and de-identified.

Among other benefits and improvements to the technical field, the methods and systems of the present teachings enable the modeling of a body surface of a patient and storing the patient's body position and pose, as well as the track and orientation of the imaging device on the patient's body surface during critical steps of the imaging protocol/workflow. This information may then be stored in the patient's electronic medical records (EMR)/picture archiving and communication system (PACS) information, and can be recalled to further examine an imaging procedure, or to improve the repeatability of a particular imaging protocol at a later time, or both. Moreover, the present teachings enable retrospective observation of a probe position in relation to the anatomy. Beneficially, and as will become clearer as the present description continues, improvements in efficiency and accuracy of imaging procedures are realized without revealing the identity of a subject.

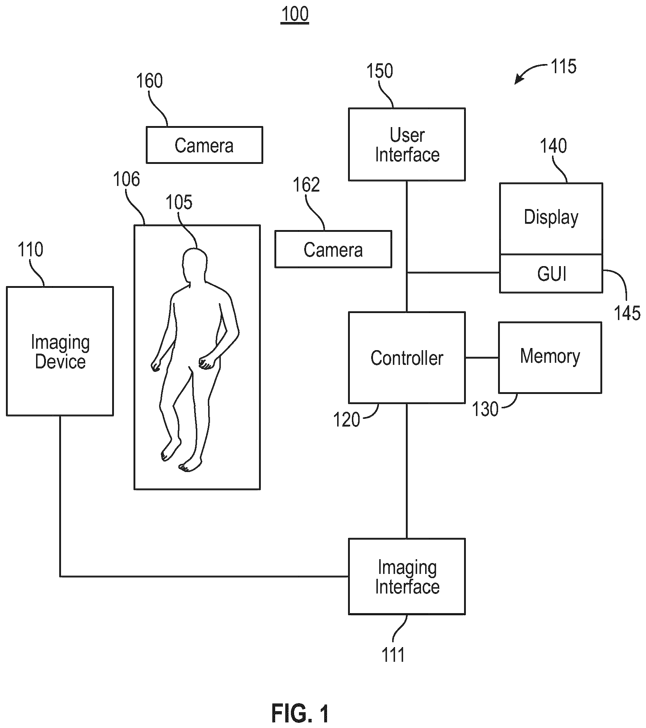

Fig. 1 is a simplified block diagram of a system 100 for segmenting an image of a region of interest of a subject, according to a representative embodiment.

Referring to Fig. 1, the system 100 includes an imaging device 110 and a computer system 115 for controlling imaging of a region of interest in a patient 105 on a table 106. The imaging device 110 may be any type of medical imaging device capable of providing an image scan of the region of interest in the patient 105. In accordance with various representative embodiments described below, the imaging device 110 is an US imaging probe or other imaging modality device compatible with the methods and systems of the present teachings described herein. As will become clearer as the present description continues, US imaging is carried out with a selected US imaging device to construct a computational model described below. Notably, the selected imaging device 110 is not typically a component of the system 100, but may be used in conjunction with the system 100 described below.

The computer system 115 receives image data from the imaging device 110, and stores and processes the imaging data according to the embodiments discussed herein. The computer system 115 includes a controller 120, a memory 130, and a display 140.

The controller 120 interfaces with the imaging device 110 through an imaging interface 111. The memory 130 stores instructions executable by the controller 120. When executed, and as described more fully below, the instructions cause the controller 120 to implement processes that include

The controller 120 is representative of one or more processing devices, and is configured to execute software instructions to perform functions as described in the various embodiments herein. The controller 120 may be implemented by field programmable gate arrays (FPGAs), application specific integrated circuits (ASICs), a general purpose computer, a central processing unit, a computer processor, a processor, a microprocessor, a microcontroller, a state machine, programmable logic device, or combinations thereof, using any combination of hardware, software, firmware, hard-wired logic circuits, or combinations thereof. Additionally, any processor provided in the controller 120 may include multiple processors, parallel processors, or both. Multiple processors may be included in, or coupled to, a single device or multiple devices.

The term "processor" as used herein encompasses an electronic component able to execute a program or machine executable instruction. References to a computing device comprising "a processor" should be interpreted to include more than one processor or processing core, as in a multi-core processor. A processor may also refer to a collection of processors within a single computer system or distributed among multiple computer systems, such as in a cloud-based or other multi-site application. The term computing device should also be interpreted to include a collection or network of computing devices each including a processor or processors. Programs have software instructions performed by one or multiple processors that may be within the same computing device or which may be distributed across multiple computing devices.

The memory 130 may include a main memory and/or a static memory, where such memories may communicate with each other and the controller 120 via one or more buses. The memory 130 stores instructions used to implement some or all aspects of methods and processes described herein. The memory 130 may be implemented by any number, type and combination of random access memory (RAM) and read-only memory (ROM), for example, and may store various types of information, such as software algorithms, which serves as instructions, which when executed by a processor cause the processor to perform various steps and methods according to the present teachings. For example, in accordance with various representative embodiments, the memory 130 that stores a (trained) first computational model comprising first instructions, and a (trained) second computational model comprising second instructions to carry out various functions described more fully below with respect to various representative embodiments. For example, the trained first computational model may be applied to predict a body type of the patient's body, and the second trained computational model may be applied to predict a pose of the patient's body.

More generally, although not necessarily, the first and second computational models may be trained and applied using another processor and memory that are not necessarily part of the computer system 115 or the system 100. In this case, after being trained, the first and second computational models may be stored as executable instructions in memory 130, for example, to be executed by a processor of the controller 120. Furthermore, updates to the first and second computational models may also be provided to the computer system 115 and stored in memory 130. Finally, and as will be apparent to one of ordinary skill in the art having the benefit of the present disclosure, according to a representative embodiment, the first and second computational models may be stored in a memory and executed by a processor that are not part of the computer system 115, but rather is connected to the imaging device 110 through an external link (e.g., a known type of internet connection). Just by way of illustration, the first and second computational models may be stored as executable instructions in a memory, and executed by a server that is remote from the imaging device 110. When executed by the processor in the remote server, the instructions cause the processor receive images from a camera; reconstruct a body surface map from the images from the camera; predict a body type based on the body surface map; predict a pose of the body; and identify on the body surface map, a portion of the body to be obscured, and a portion of the body to be imaged during the medical imaging.

The various types of ROM and RAM may include any number, type and combination of computer readable storage media, such as a disk drive, flash memory, an electrically programmable read-only memory (EPROM), an electrically erasable and programmable read only memory (EEPROM), registers, a hard disk, a removable disk, tape, compact disk read only memory (CD-ROM), digital versatile disk (DVD), floppy disk, Blu-ray disk, a universal serial bus (USB) drive, or any other form of storage medium known in the art. The memory 130 is a tangible storage medium for storing data and executable software instructions, and is non-transitory during the time software instructions are stored therein. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The memory 130 may store software instructions and/or computer readable code that enable performance of various functions. The memory 130 may be secure and/or encrypted, or unsecure and/or unencrypted. Examples of such media are noted above, and include non-transitory media such as computer memory devices that store information in a format that is readable by a computer or data processing system. Memory 130 is an example of computer-readable storage media, and should be interpreted as possibly being multiple memories or databases. The memory 130, for example, may comprise multiple memories local to the system 100, and/or distributed amongst multiple computer systems or computing devices.

The controller 120 illustratively includes or has access to an artificial intelligence (AI) engine, which may be implemented as software that provides artificial intelligence (e.g., body shape, body pose, imaging device (e.g., US probe) position and orientation relative to the anatomy) and applies machine-learning described herein. The AI engines contemplated include for example different/cascade models for different tasks of the present teachings. These AI engines provide the first and second computational models described below, may reside in any of various components in addition to or other than the controller 120, such as the memory 130, an external server, and/or a cloud, for example. When the AI engine is implemented in a cloud, such as at a data center, for example, the AI engine may be connected to the controller 120 via the internet using one or more wired and/or wireless connection(s). The AI engine may be connected to multiple different computers including the controller 120, so that the artificial intelligence and machine-learning described below in connection with various representative embodiments are performed centrally based on and for a relatively large set of medical facilities and corresponding subjects at different locations. Alternatively, the AI engine may implement the artificial intelligence and the machine-learning locally to the controller 120, such as at a single medical facility or in conjunction with a single imaging device 110.

The interface 150 may include a user and/or network interface for providing information and data output by the controller 120 and/or the memory 130 to the user and/or for receiving information and data input by the user. That is, the interface 150 enables the user to enter data and to control or manipulate aspects of the processes described herein, and also enables the controller 120 to indicate the effects of the user's control or manipulation. The interface 150 may include one or more of ports, disk drives, wireless antennas, or other types of receiver circuitry. The interface 150 may further connect one or more user interfaces, such as a mouse, a keyboard, a mouse, a trackball, a joystick, a microphone, a video camera, a touchpad, a touchscreen, voice or gesture recognition captured by a microphone or video camera, for example.

The display 140 may be a monitor such as a computer monitor, a television, a liquid crystal display (LCD), a light emitting diode (LED) display, a flat panel display, a solid-state display, or a cathode ray tube (CRT) display, or an electronic whiteboard, for example. The display 140 may also provide a graphical user interface (GUI) 145 for displaying and receiving information to and from the user.

Finally, the system 100 includes a first camera 160 and a second camera 162. It is noted that the inclusion of two cameras is merely illustrative, and the present teachings contemplate the use of one camera or more than two cameras. Notably, the larger the number of cameras that are used in the system, the higher the resolution of the body surface images, and the more accurate are the predicted body type, the body pose, and the position of the imaging device 110.

The first and second cameras 160, 162 may be mounted on the wall or on the ceiling of the examination room, and enable the entire imagination room to be scanned without missing, for example, portions of the patient's body or the movements of the clinician operating the imaging device 110.

Each of the first and second cameras 160, 162 is illustratively a so-called 3D camera, which is an imaging device that enables the perception of depth in images to replicate three dimensions as experienced through human binocular vision. Notably, the first and second cameras 160, 162 are 3D cameras that may have a plurality of lenses to record multiple points of view, or a single lens that shifts its position.

As will be described more fully below, the first and second cameras 160, 162 are used to create a body surface reconstruction of the patient. Moreover, the first and second cameras enable the recognition of different features of the patient's body, the clinician (e.g., sonographer), the imaging device 110, and the examination room. The recognition of different pieces of the body allows for the connection of these body parts to a specific imaging protocol, and the type of exam that needs to be carried out. As described more fully below, application of the first and second computational models uses the different pieces of the body to predict a body shape of the patient 105, and recognize the location and orientation of the imaging device 110 and the pose of the patient. Again, the prediction of the pose of the patient 105 and the position and orientation of the imaging device 110 may be done while preserving the anonymity of the patient 105.

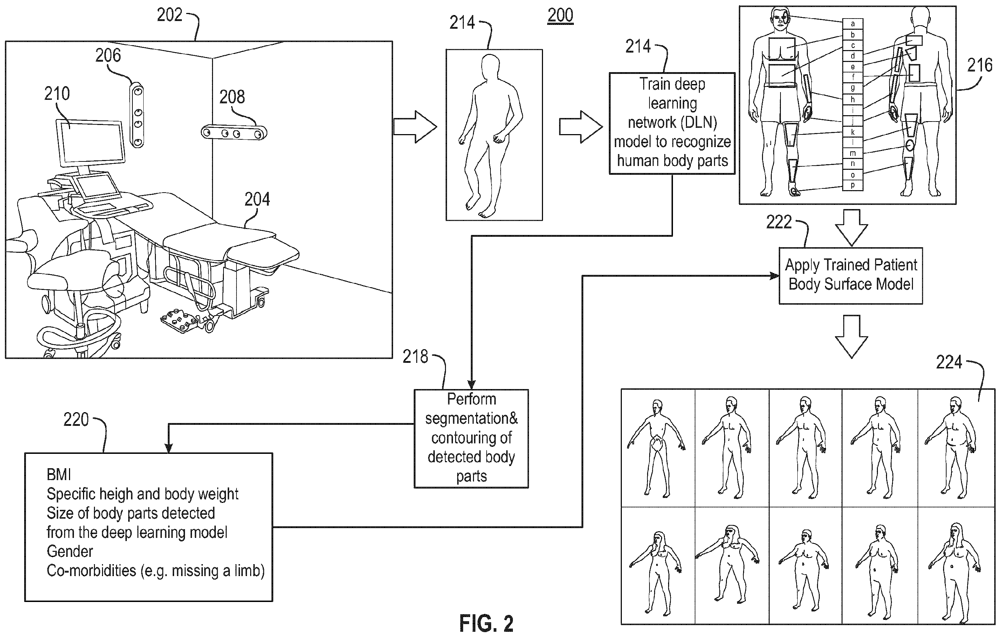

Fig. 2 is a simplified flow diagram of a method 200 for capturing body surface maps and predicting a body type in accordance with a representative embodiment. Various components, aspects and details of the method 200 are common to those described in connection with the system 100 and may not be repeated to avoid obscuring the presently described embodiments.

An examination room 202 comprises an examination table 204, first and second cameras 206, 208, and a US imaging system 210. The first and second cameras 206, 208 are illustratively mounted on the walls and capture 3D images of the patient (not shown in Fig. 2), and may also capture 3D images of the sonographer (not shown in Fig. 2). Notably, in addition to the first and second cameras 206, 208, or instead of one of the 3D cameras, a conventional camera may be provided to capture real time images of the patient. These images may be used, for example, to provide a real-time image of various elements of in the examination room 202, such as, for example, portions of the patient that do not necessarily need to be obscured to preserve patient anonymity or that do not include sensitive areas of the patient.

As alluded to above, and as described more fully below, the images gathered from the first and second cameras 206, 208 are processed as described more fully below, and can be used for many benefits. For example, the processed images can be used to monitor workflow of the imaging procedure by personnel not in the examination room 202 to provide an update to the patients waiting to be examined. Similarly, the processed images may be used to facilitate a subsequent, repeat imaging procedure on the same patient by providing the captured pose of the patient over time, and the location and motion of the US imaging probe (not shown in Fig. 2). As will be appreciated by one of ordinary skill in the art, the processed images based on the images from the first and second cameras 206, 208 will improve monitoring of the status of the current imaging procedure, and improve the efficiency and accuracy of a subsequent imaging procedure of the patient, whether a repeat imaging procedure, or another imaging procedure that may benefit from the knowledge of the pose of the patient's body, or the location and motion of the US probe, or both.

The method 200 continues with the gathering of a 3D image 212 of the patient, which is illustratively a body surface map of the particular. Notably, the entire image of the patient is illustratively blurred at this point to preserve anonymity. In such case, the camera feed will still focus on the body parts being scanned during the imaging exam, but the saved information will be in a non-identifiable format. Alternatively, the image 212 may not initially be obscured completely, or at all, with the obscuring being done after processing the image 212. Notably, the unobscured areas are relevant to the body part being scanned and therefore the areas where the camera feed is collected during the imaging exam. The rest of the body remains obscured; hence, non-identifiable. In this portion of the method 200, the depth map of the patient is saved for further processing as described below.

In certain embodiments, the camera feed may be acquired either in the examination room 202, or in the waiting room before the commencement of the imaging procedure. In such embodiments, one of the first and second cameras 206, 208 may be used to acquire a baseline image of the patient, and the other of the first and second cameras 206, 208 (or additional cameras) in the examination room 202 is used to collect image data for which obscuring of the image (sometimes referred to as de-identification) is carried out, and the exam is being monitored. The first computational model will de-identify initially (and roughly) the body parts that do not belong to the scanning protocol and patient's identify. Notably, as used herein, de-identification includes blurring over the camera images. For example, the body surface map can be acquired in the exam room, while the patient waits for the sonographer to review the exam and scanning protocol. Once the patient's body surface area is modelled by the method 200 and the scanning protocol is selected, clinicians can review the imaging workflow before entering the exam room. In certain cardiac imaging exams sonographers can benefit from knowing a priori if the patient has a high rib cage (barrel chest) so they can select different probes (i.e. next generation imaging probes with smaller foot prints to fit the rib space) and modify the imaging protocol as needed. In other clinical applications, such as lower limbs imaging, sonographers can select different probes with higher imaging frequencies if patients have very thin and skinny legs, for example.

As will be appreciated by one of ordinary skill in the art, the processed images based on the images from the first and second cameras will improve monitoring of the status of the current imaging procedure, and improve the efficiency and accuracy of a subsequent imaging procedure of the patient, whether a repeat imaging procedure, or another imaging procedure that may benefit from the knowledge of the pose of the patient's body, or the location and motion of the US probe, or both, while maintaining the privacy of the patient.

At 214, the first computational model used to identify body parts is trained. The first computational model may be a deep learning network (DLN) model.

In deep learning, which is a subfield of machine-learning, the inner parameters inside the computational model are organized into successively connected layers, where each layer produces increasingly meaningful output as input to the next layer, until the last layer which produces the final output.

Deep learning layers useful in accordance with the present teachings are typically implemented as so-called neural networks, that is, layers are comprised of a set of nodes, each representing an output value and a prescription on how to compute this output value from the set of output values of the previous layer's nodes. The prescription being a weighted sum of transformed output values of the previous layer's nodes, each node only needs to store the weights. The transformation function is the same for all nodes in a layer and is also called activation function. There are a limited number of activation functions that are used today. A particular way to set which previous layer's nodes provide input to a next layer's node is convolution. Neural networks based on convolution are called convolutional neural networks (CNNs).

Thus, in the learning phase or so-called training, for each example, the output of the final layer is computed. Outputs for all examples are compared with the desired outputs by way of the objective function. The output of the objective function, the so-called loss, is used as a feedback signal to adjust the weights such that the loss is reduced. The adjustment, i.e. which weights to change and by how much, is computed by the central algorithm of deep learning, so-called backpropagation, which is based on the fact that the weighted sums that connect the layer nodes are functions that have simple derivatives. The adjustment is iterated until the loss reaches a prescribed threshold or no longer changes significantly.

A deep learning network thus can be stored (e.g., in memory 130) as a topology that describes the layers and activation functions and a (large) set of weights (simply values). A trained network is the same, only the weights are now fixed to particular values. Once the network is trained, it is put to use, that is, to predict output for new input for which the desired output is unknown.

In the present teaching, the first computational model is stored as instructions to provide the machine-learning algorithm, such as a convolutional neural-network deep learning algorithm.

The training of the first computational model at 214 includes the use of ground truth data of the patient as shown at 216, hence, bounding boxes indicating body parts. The output is expected to be bounding boxes of identified body parts and corresponding counting/segmentations. Specifically, as shown, using ground truth data from the patient, the first computational model learns to identify certain anatomical elements (e.g., the chest area, the arm(s), the leg(s), the shoulder and the back) of the patient based on the surface body map gathered at 212. The identification of the selected body parts from the patient's body maps enables the blurring/obscuring of parts of the patient's body, which provides certain benefits. For example, the upcoming exam type to be carried out on the patient, and the corresponding imaging protocols can be extracted and retrieved from the PACS. The body parts that are not included in the scanning protocol will be obscured during the camera feed while the patient is being examined.

Once the first computational model is trained, a segmentation and contouring of the detected body parts from the surface body map are carried out at 218 using known methods. At 220 the segmentation and contouring data are added to other ground truth data comprising non-imaging clinical data including, but not limited to a body mass index (BMI), a size of body parts, a gender and co-morbidities. The data from 218 and 220 are then provided to the first computational model. This first computational model may include elements described in "A Framework for Analyzing the Whole Body Surface from a Single View" to Piccirilli, et al. (PLoS One. 2017 Jan 3;12(1):e0166749. doi: 10.1371/journal.pone.0166749. PMID: 28045895; PMCID: PMC5207503). Based on the segmentation and contouring data gathered from the body surface map at 212 and the clinical data, the trained first computational model predicts the body shape of the person based on a number of body types shown at 224. As alluded to above, the input parameters to the first computational model comprise patient-specific data, which may comprise imaging and non-imaging data. Specifically, based on the data from the body surface map and the clinical data as input parameters, the first computational model identifies patterns, which are compared to previously obtained patterns corresponding to known body types. In an embodiment, the first computational model applies weights to the various input parameters provided thereto to improve the accuracy of the predicted body type at 224. Different body types are modelled based on the parameters in 220 and the body parts extracted from 218, and based on the modeling, the body type closest to the patient's body surface map is selected. Accordingly, based on the body surface map at 212, the first computational model provides not only a predicted body shape, but also a predicted body habitus. As described more fully below, the predicted body shape and habitus are useful to the identification of body parts and joints, which are useful in predicting the pose of the patient over time, and in predicting the location of the US probe (or other imaging device 110) on the body over time.

As alluded to above and as described more fully below, body parts are identified in method 200 by training the first computational model on body surface areas of different shapes. The first computational model can learn body parts such as neck area, chest, arm, abdomen, and any other segments of the patient body that can be ideally part of an ultrasound exam. The de-identification is done by masking and blurring all body parts that are not included in the imaging protocol. However, based on the imaging protocol, the first computational model will identify and obscure portions of to be obscured to preserve patient anonymity. As such, the first and second cameras 206, 208 are set up to observe and dynamically monitor that area of the patient's body surface during the imaging exam. The areas that remain unmasked/unblurred may be the areas under imaging investigation.

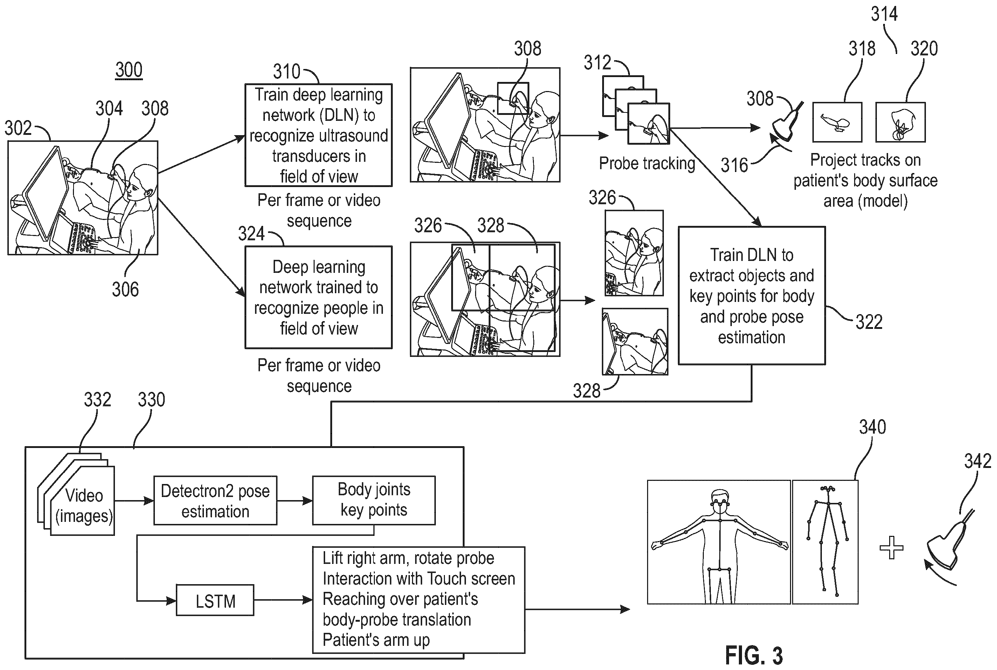

Fig. 3 is a simplified flow diagram of a method 300 for predicting a body pose and a location of an US probe in accordance with a representative embodiment. Various components, aspects and details of the method 300 are common to those described in connection with the method 200 and the system 100 and may not be repeated to avoid obscuring the presently described embodiments.

As will become clearer as the present description continues, the method 300 trains and applies a second computational model used to predict the pose of a body over time, the position of the US probe, and the orientation of the US probe, all over time. The second computational model may be implemented as a DLN, for example. For ease of description the second computational model is presented as two DLNs, with one trained to predict the pose of the body, and the other to predict the pose of the US probe. However, a single DLN to predict both body pose, and US probe position and orientation is contemplated. As with the first computational model described above, the currently described second computational model can be stored (e.g., in memory 130) as a topology that describes the layers and activation functions and a (large) set of weights (simply values). A trained network of the second computational model is the same, only the weights are now fixed to particular values. Once the network is trained, it is put to use, that is, to predict output for new input for which the desired output is unknown.

The method 300 begins at 302, which shows a patient 304, a clinician 306 and a US probe 308 being applied to the patient 304.

At 310, the method continues with the training of the second computational model to recognize the US probe 308. The architecture of the second computational model may be one of a number of known AI architectures, including Detectron or EfficientDet useful in object and key points detection. In essence, these platforms are trained to extract a pose, location and orientation of an object (e.g., a body part and US probe). During a learning phase, training parameters are modified to make ensure acceptable accuracy in model prediction. Typical parameters related to the training of the illustrative DLN of the second computational model include, but are not limited to filter size, number of convolutional layers and number of hidden layers, stride size, Dropout, batch normalization, activation function, optimizers, learning rate, number of epochs, momentum, batch size. Moreover, the pose prediction may also be weighted based on the user's actions corresponding to the scanning protocol.

At 312, frames of video gathered from the imaging device are shown. These frames are sequential in time, and are used to track the movement and orientation 316 of the US probe 308 and to predict future movement using the second computational model. Furthermore, as shown at 314 tracks 318, 320 of the US probe 308 are projected on the modeled body surface area of the patient under investigation. As alluded to above, the orientation, location and track of the US probe 308 may be stored and later referenced in a subsequent imaging procedure. Moreover, the saved orientation, location and tracking of the US probe 308 may be referenced by a radiologist reviewing the scans of the imaging procedure.

The probe identification and the corresponding bounding boxes coordinates carried out at 312 is provided at 322 for training of the second computational model to extract objects and key points for body and probe pose.

At 324, the second computational model is trained to recognize people in a field of view. Specifically, as shown in frame 326 the patient is recognized, and in frame 328, the clinician (sonographer) is recognized by the second computational model. These recognized people are then provided at 322 to train key points for body pose. Notably, the body pose gathered for inclusion at 322 can be received from method 200 as described above.

At 330, zooming into to the specific workflow is carried out to complete the training of the second computational model for pose simulation and action prediction. Video images 332 are the input to the second computational model using, in this example, the Detectron2 platform for pose estimation. Specifically, key points (e.g., body points) are weighted for the second computational model to predict the body pose and position of the presently described representative embodiment. Alternatively, or additionally, the body pose and position may be achieved according to the teachings of commonly-owned U.S. Provisional Application No. 63/250,511 entitled "System for Estimating the Pose of a Subject" and filed on September 30, 2021.

As shown at 330, the second computational model is applied, which may be a Long-Short-Term-Memory (LSTM) recurrent neural network. As is known, LSTM is a recurrent neural network that is used to learn temporal information. In the presently described representative embodiment, the LSTM is applied to learn sequences of actions present in video/frame sequences gathered by first and second cameras 206, 208, or real-time image cameras. LSTM's are suitable for time series data and events. In presently described representative embodiment, the LSTM is the engine used to take key points as inputs and predict user and patient action/motion, along with transducer pose and motion throughout the imaging protocol/exam.

After application of the second computational model at 330, the pose estimation of both the clinician and the patient are provided at 340. Moreover, application of the second computational model provides a prediction of joint locations and patient action. Finally, at 342, the orientation of the US probe 308 and a prediction of its movement in the US procedure are also provided by the second computational model. As noted above, the prediction of the pose of a patient, and the prediction of the location and orientation of the US probe 308 over time is realized. Among other benefits, the second computational model allows for scans to be stored and referenced in a future US imaging procedure, or by the radiologist, or both,

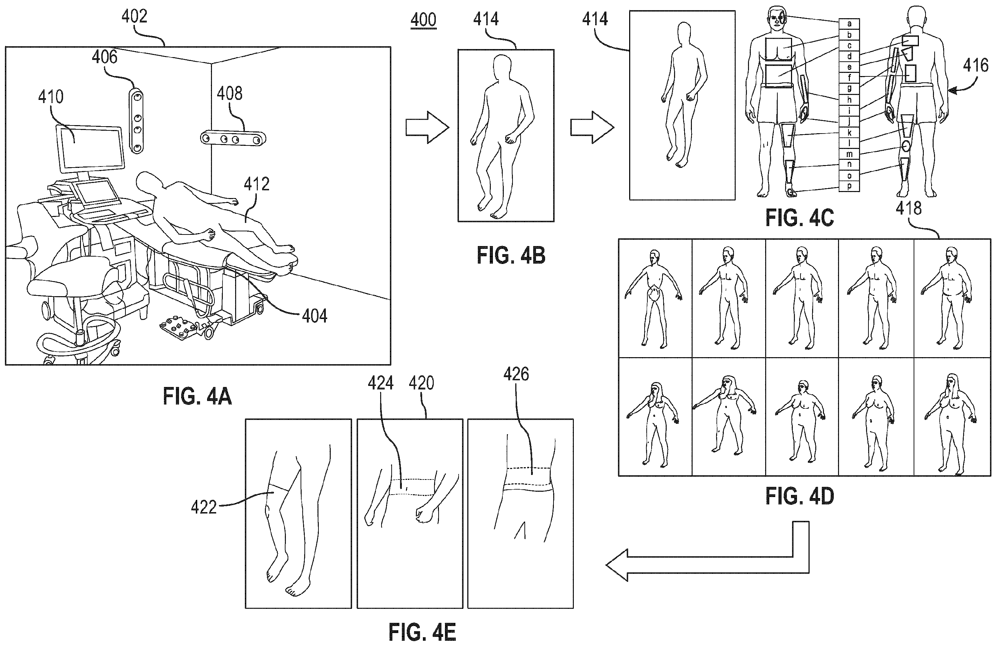

Figs. 4A-4E is a flow diagram of an ultrasound imaging method 400 using computational models according to a representative embodiment. Various components, aspects and details of the ultrasound imaging method 400 are common to those described in connection with the system 100, and methods 200, 300 and may not be repeated to avoid obscuring the presently described embodiments.

An examination room 402 comprises an examination table 404, first and second cameras 406, 408, and a US imaging system 410. A patient 412 is situated on the examination table. The first and second cameras 406, 408 are illustratively mounted on the walls and capture 3D images of the patient 412, and may also capture 3D images of the sonographer (not shown in Fig. 4). Again, in addition to the first and second cameras 406, 408, or instead of one of the 3D cameras, a conventional camera may be provided to capture real time images of the patient. These images may be used, for example, to provide a real-time image of various elements of in the examination room 402, such as, for example, portions of the patient that do not necessarily need to be obscured to preserve patient anonymity or that do not include sensitive areas of the patient.

As described above, the images gathered from the first and second cameras 406, 408 are processed as described more fully below, and can be used for many benefits. For example, the processed images can be used to monitor workflow of the imaging procedure by personnel not in the examination room 402 to provide an update to the patients waiting to be examined. Similarly, the processed images may be used to facilitate a subsequent, repeat imaging procedure on the same patient by providing the captured pose of the patient over time, and the location and motion of the US imaging probe (not shown in Fig. 4). As will be appreciated by one of ordinary skill in the art, the processed images based on the images from the first and second cameras will improve monitoring of the status of the current imaging procedure, and improve the efficiency and accuracy of a subsequent imaging procedure of the patient, whether a repeat imaging procedure, or another imaging procedure that may benefit from the knowledge of the pose of the patient's body, or the location and motion of the US probe, or both.

The ultrasound imaging method 400 continues with the gathering of a 3D image 414 of the patient 412, which is illustratively a body surface map of the particular patient. Notably, the entire image of the patient is illustratively obscured at this point to preserve anonymity. In such case, the camera feed will still focus on the body parts being scanned during the imaging exam, but the saved information will be in a non-identifiable format. Alternatively, the image 414 may not initially be obscured completely or at all, with the obscuring being done after processing the image 414, such as described in connection with Fig. 4E below. Notably, the unobscured areas are relevant to the body part being scanned and therefore the areas where the camera feed is collected during the imaging exam. The rest of the body remains obscured; hence, non-identifiable. In this portion of the ultrasound imaging method 400, the depth map of the patient is saved for further processing as described below.

In certain embodiments, the camera feed may be acquired either in the examination room 402, or in the waiting room before the commencement of the imaging procedure. In such embodiments, one of the first and second cameras 406, 408 may be used to acquire a baseline image of the patient, and the other of the first and second cameras 406, 408 (or additional cameras) in the examination room 402 may be used to acquire image data for which obscuring of the image (sometimes referred to as de-identification) is carried out, and the exam is being monitored. For example, the body surface map can be acquired in the exam room, while the patient waits for the sonographer to review the exam and scanning protocol. Once the patient's body surface area is modelled by the ultrasound imaging method 400 and the scanning protocol is selected, clinicians can review the imaging workflow before entering the exam room. In certain cardiac imaging exams sonographers can benefit from knowing a priori if the patient has a high rib cage (barrel chest) so they can select different probes (i.e. next generation imaging probes with smaller foot prints to fit the rib space) and modify the imaging protocol as needed. In other clinical applications, such as lower limbs imaging, sonographers can select different probes with higher imaging frequencies if patients have very thin and skinny legs, for example.

As will be appreciated by one of ordinary skill in the art, the processed images based on the images from the first and second cameras will improve monitoring of the status of the current imaging procedure, and improve the efficiency and accuracy of a subsequent imaging procedure of the patient, whether a repeat imaging procedure, or another imaging procedure that may benefit from the knowledge of the pose of the patient's body, or the location and motion of the US probe, or both.

At 416 and 418, the first computational model is applied to the 3D image 414 of the patient. As discussed above, segmentation and contouring of the detected body parts from the surface body map are carried out. These data are added to other ground truth data comprising clinical data including, but not limited to a body mass index (BMI), a size of body parts, a gender and co-morbidities. The data from are then provided to the first computational model, (trained DLN model or generic body surface computational model). Based on data gathered from the body surface map (image 414) and the clinical data, the trained first computational model predicts the body shape of the person based on a number of body types shown at 424. As described above, based on the data from the body surface map (image 414) and the clinical data, the first computational model applies weights to the various parameters provided to thereto to improve the accuracy of the predicted body type. Accordingly, based on the body surface map (image 414), the first computational model provides not only a predicted body shape, but also a predict body habitus. The predicted body shape and habitus are useful to the identification of body parts and joints, which are useful in predicting the pose of the patient over time and in predicting the location of the US probe (or other imaging device 110) on the body over time.

Furthermore, as shown at 420, different portions of the body may be selected for viewing by the first computational model. Specifically, based on the imaging protocol, only the portion of the body from the body surface map (image 414) to be imaged in the imaging procedure may be provided. Moreover, specific anatomical regions 422, 424, 426 may be isolated by the first computational model to be shown and saved in the imaging procedure. The regions outside of the specific anatomical regions 422, 424, 426 may be obscured to preserve patient anonymity.

Accordingly, during operation of the ultrasound imaging method 400, the first and second cameras 406, 408 monitor the imaging exam by first saving the patient body surface map (image 414) with respective body position during the imaging exam (i.e., a depth map with patient decubitus) while DICOM images are being captured.

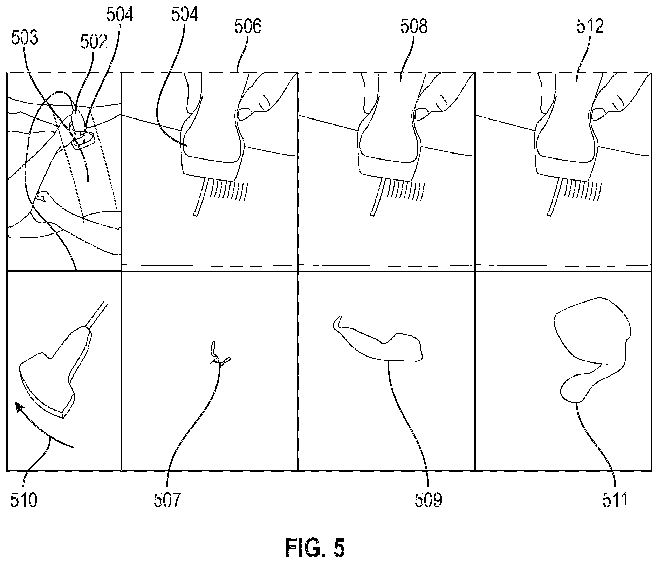

Fig. 5 shows the orientation and movement of a US imaging probe 504 according to a representative embodiment. Various components, aspects and details of the components of the US imaging probe and its orientation relative to an anatomical body part, and its function of are common to those described in connection with the system 100, and methods 200, 300, 400 and may not be repeated to avoid obscuring the presently described embodiments.

At 502, the US imaging probe 504 is disposed over an abdomen 503 in a selected orientation relative to the abdomen 503. As alluded to above, the body surface map and selected body pose described above in connection with Figs. 2 and 4 are saved in the system 100, so that the location and orientation of the US imaging probe 504 can be determined using the second computational model. The direction of movement 510 of the imaging probe 504 is merely illustrative. Moreover, in addition to determining the relative orientation of the imaging probe 504, the path of the US imaging probe 504 can be tracked and saved, again using the second computational model. As such, frame 506 shows a path 507 of the US imaging probe 504; frame 508 shows a path 509 of the US imaging probe 504; and frame 512 shows a path 511 of the US imaging probe 504. Notably, the tracks of the US imaging probe 504 may also be projected over the reconstructed body surface map. Again, among other benefits this facilitates the reproduction of an imaging procedure at a later time, and may be useful to the radiologist reading the scans from the recent imaging procedure.

As noted above, the orientation and location of the US imaging probe over time in an imaging procedure can be saved in memory 130 and reviewed by the sonographer in a subsequent imaging procedure of the same patient, or used by the radiologist during review of the scan, or both. Accordingly using the body pose and body surface map determined using the first computational model, the track of the US imaging probe 504 on patient body surface area map within imaging protocol and is captured, and based on the cameral feed portions of the camera feed can be obscured to maintain anonymity.

The second computational model in turn identifies and records probe tracks, and body position and probe orientation relative to the body in the camera feed and DICOM feed. Specifically, by registering the body surface map and body pose using the first computational model to the cameral feed during the imaging procedure, body parts included in the protocol remain unchanged in the camera stream, and all other body parts are replaced by the registered model. The resulting image is one comprising essentially a body model representing the patient with only the relevant body part unmasked. Such a composite camera frame is stored alongside the DICOM images and the probe tracks, and a final representation of the probe pose projected onto the reconstructed patient body surface area.

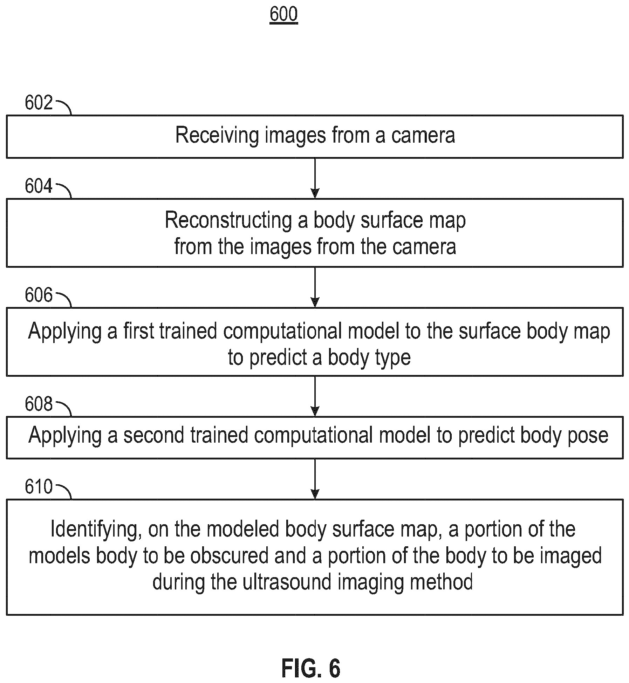

Fig. 6 is a simplified flow diagram showing a method 600 of performing ultrasound imaging using a trained computational model, according to a representative embodiment. Various components, aspects and details of the components of the US imaging probe and its orientation relative to an anatomical body part, and its function of are common to those described in connection with the system 100, and methods 200, 300, 400 and 500 may not be repeated to avoid obscuring the presently described embodiments.

At 602, the method 600 comprises receiving images from a camera.

At 604, the method comprises reconstructing a body surface map from the images from the camera.

At 606, the method 600 comprises applying a first trained computational model to the body surface map to predict a body type.

At 608, the method 600 comprises applying a second trained computational model to predict a pose of the body.

At 610, the method comprises identifying, on the body surface map, a portion of the body to be obscured, and a portion of the body to be imaged during the ultrasound imaging method.

Although methods, systems and components for creating composite camera images for body surface area modeling and deidentification of patients, as well as predicting body pose and ultrasound imaging probe track and orientation in ultrasound imaging exams have been described with reference to several exemplary embodiments, it is understood that the words that have been used are words of description and illustration, rather than words of limitation. Although developing adaptable predictive analytics has been described with reference to particular means, materials and embodiments, developing adaptable predictive analytics is not intended to be limited to the particulars disclosed; rather developing adaptable predictive analytics extends to all functionally equivalent structures, methods, and uses such as are within the scope of the appended claims.

The illustrations of the embodiments described herein are intended to provide a general understanding of the structure of the various embodiments. The illustrations are not intended to serve as a complete description of all of the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

Although specific embodiments have been illustrated and described herein, it should be appreciated that any subsequent arrangement designed to achieve the same or similar purpose may be substituted for the specific embodiments shown. This disclosure is intended to cover any and all subsequent adaptations or variations of various embodiments. Combinations of the above embodiments, and other embodiments not specifically described herein, will be apparent to those of skill in the art upon reviewing the description.

In addition, in the foregoing Detailed Description, various features may be grouped together or described in a single embodiment for the purpose of streamlining the disclosure. This disclosure is not to be interpreted as reflecting an intention that the claimed embodiments require more features than are expressly recited in each claim. Rather, as the following claims reflect, inventive subject matter may be directed to less than all of the features of any of the disclosed embodiments. Thus, the following claims are incorporated into the Detailed Description, with each claim standing on its own as defining separately claimed subject matter.

The preceding description of the disclosed embodiments is provided to enable any person skilled in the art to practice the concepts described in the present disclosure. As such, the above disclosed subject matter is to be considered illustrative, and not restrictive, and the appended claims are intended to cover all such modifications, enhancements, and other embodiments which fall within the scope of the present disclosure. Thus, to the maximum extent allowed by law, the scope of the present disclosure is to be determined by the broadest permissible interpretation of the following claims and their equivalents and shall not be restricted or limited by the foregoing detailed description.

## Claims

1. A method (600) of providing workflow imaging of a patient for performing an ultrasound imaging procedure, the method (600) comprising:
receiving images from a camera (602);
reconstructing a body surface map from the images from the camera (604), wherein the body surface map comprises a digital contour map of a surface area of the patient's body;
applying a first trained computational model to the body surface map to predict a body type of the patient from a number of known body types (606);
applying a second trained computational model to the received images to predict a pose of the body (608) based on the predicted body type; and
identifying, on the body surface map, a portion of the body to be obscured, and a portion of the body to be imaged (610) during the ultrasound imaging procedure by registering the body surface map and the predicted pose of the body of the received images.

2. The method (600) of claim 1, further comprising:
applying the second trained computational model to track a position of an ultrasound imaging probe (504) during an ultrasound imaging procedure.

3. The method (600) of claim 2, further comprising applying the second trained computational model to predict an orientation (316) of the ultrasound imaging probe (504) relative to the predicted pose of the body.

4. The method (600) of claim 3, wherein applying the second trained computational model further comprises identifying anatomical parts.

5. The method (600) of claim 2, further comprising coupling the tracked position of the ultrasound imaging probe (504) to the body surface map, and saving the tracked position relative to the body surface map.

6. The method (600) of claim 1, wherein the applying the first trained computational model to predict the body type further comprises selecting the body type that is closest to the patient's body surface map.

7. The method (600) of claim 2, wherein the applying the second trained computational model to track a position of the ultrasound imaging probe (504) further comprises predicting movement (510) of the ultrasound imaging probe (504) based on a plurality of video frames.

8. The method (600) of claim 6, wherein the applying the first trained computational model further comprises providing ground truth data to the first trained computational model to predict the body type, and wherein preferably the ground truth data comprise non-imaging data.

9. A system (100) for providing workflow imaging of a patient for a medical imaging procedure, comprising:
a camera adapted to capture a three-dimensional (3D) an image (212);
a memory (130) adapted to store a first computational model comprising first instructions, and a second computational model comprising second instructions; and
a processor, wherein the first instructions or the second instructions, when executed by the processor, cause the processor to:
- receive images from the camera;
- reconstruct a body surface map from the images from the camera, wherein the body surface map comprises a digital contour map of a surface area of the patient's body;
- apply the first trained computational model to the body surface map to predict a body type of the patient from a number of known body types;
- apply the second trained computational model to the received images to predict a pose of the body based on the predicted body type; and
- identify, on the body surface map, a portion of the body to be obscured, and a portion of the body to be imaged during the medical imaging procedure by registering the body surface map and the predicted pose of the body of the received images.

10. The system (100) of claim 9, wherein the second instructions, when executed by the processor cause the processor to predict an orientation (316) of an ultrasound imaging probe (504) relative to the predicted pose of the body.

11. The system (100) of claim 10, wherein the second instructions, when executed by the processor further causes the processor to identify anatomical parts.

12. The system (100) of claim 9, wherein the second instructions, when executed by the processor further cause the processor to: track a position of an ultrasound imaging probe (504) during an imaging protocol, couple the tracked position of the ultrasound imaging probe (504) to the body surface map, and save the tracked position relative to the body surface map.

13. The system (100) of claim 9, wherein the second instructions, when executed by the processor further cause the processor to: track a position of an ultrasound imaging probe (504) during an imaging protocol, and predict movement (510) of an ultrasound imaging probe (504) based on a plurality of video frames.

14. The system (100) of claim 9, wherein the first instructions, when executed by the processor, cause the processor to predict the body type based on ground truth data, and wherein preferably the ground truth data comprise non-imaging data.

15. A tangible, non-transitory computer readable medium that stores a first computational model comprising first instructions and a second computational model comprising second instructions, which when executed by a processor, cause the processor to perform the steps of the method of any of claims 1-8.

## Patentansprüche

1. Verfahren (600) zum Bereitstellen von Workflow-Bildgebung eines Patienten zum Durchführen einer Ultraschallbildgebungsprozedur, wobei das Verfahren (600) Folgendes umfasst:
Empfangen von Bildern von einer Kamera (602);
Rekonstruieren einer Körperoberflächenkarte aus den Bildern von der Kamera (604), wobei die Körperoberflächenkarte eine digitale Konturkarte eines Oberflächenbereichs des Körpers des Patienten umfasst;
Anwenden eines ersten trainierten Rechenmodells auf die Körperoberflächenkarte, um aus einer Reihe bekannter Körpertypen (606) den Körpertyp des Patienten vorherzusagen;
Anwenden eines zweiten trainierten Rechenmodells auf die empfangenen Bilder, um basierend auf dem vorhergesagten Körpertyp eine Körperhaltung (608) vorherzusagen; und
Identifizieren, auf der Körperoberflächenkarte, eines Abschnitts des Körpers, der verdunkelt werden soll, und eines Abschnitts des Körpers, der während der Ultraschallbildgebungsprozedur abgebildet (610) werden soll, durch Registrieren der Körperoberflächenkarte und der vorhergesagten Körperhaltung auf den empfangenen Bildern.

2. Verfahren (600) nach Anspruch 1, weiter umfassend:
Anwenden des zweiten trainierten Rechenmodells zum Verfolgen einer Position einer Ultraschallbildgebungssonde (504) während einer Ultraschallbildgebungsprozedur.

3. Verfahren (600) nach Anspruch 2, weiter umfassend Anwenden des zweiten trainierten Rechenmodells zum Vorhersagen einer Orientierung (316) der Ultraschallbildgebungssonde (504) in Bezug auf die vorhergesagte Körperhaltung.

4. Verfahren (600) nach Anspruch 3, wobei Anwenden des zweiten trainierten Rechenmodells weiter Identifizieren anatomischer Teile umfasst.

5. Verfahren (600) nach Anspruch 2, weiter umfassend Koppeln der verfolgten Position der Ultraschallbildgebungssonde (504) an die Körperoberflächenkarte und Speichern der verfolgten Position in Bezug auf die Körperoberflächenkarte.

6. Verfahren (600) nach Anspruch 1, wobei Anwenden des ersten trainierten Rechenmodells zum Vorhersagen des Körpertyps weiter Auswählen des Körpertyps umfasst, der der Körperoberflächenkarte des Patienten am nächsten kommt.

7. Verfahren (600) nach Anspruch 2, wobei Anwenden des zweiten trainierten Rechenmodells zum Verfolgen einer Position der Ultraschallbildgebungssonde (504) weiter Vorhersagen von Bewegung (510) der Ultraschallbildgebungssonde (504) basierend auf einer Vielzahl von Videobildern umfasst.

8. Verfahren (600) nach Anspruch 6, wobei das Anwenden des ersten trainierten Rechenmodells weiter Bereitstellen von Grundwahrheitsdaten für das erste trainierte Rechenmodell zum Vorhersagen des Körpertyps umfasst, wobei die Grundwahrheitsdaten vorzugsweise Nicht-Bildgebungsdaten umfassen.

9. System (100) zum Bereitstellen von Workflow-Bildgebung eines Patienten für eine medizinische Bildgebungsprozedur, umfassend:
eine Kamera, die zum Aufnehmen eines dreidimensionalen (3D) Bildes (212) angepasst ist;
einen Speicher (130), der zum Speichern eines ersten Rechenmodells, das erste Anweisungen umfasst, und eines zweiten Rechenmodells, das zweite Anweisungen umfasst, angepasst ist; und
einen Prozessor, wobei die ersten Anweisungen oder die zweiten Anweisungen, wenn sie vom Prozessor ausgeführt werden, bewirken, dass der Prozessor:
- Bilder von der Kamera empfängt;
- aus den Bildern von der Kamera eine Körperoberflächenkarte rekonstruiert, wobei die Körperoberflächenkarte eine digitale Konturkarte eines Oberflächenbereichs des Körpers des Patienten umfasst;
- das erste trainierte Rechenmodell auf die Körperoberflächenkarte anwendet, um aus einer Reihe bekannter Körpertypen einen Körpertyp des Patienten vorherzusagen;
- das zweite trainierte Rechenmodell auf die empfangenen Bilder anwendet, um basierend auf dem vorhergesagten Körpertyp eine Körperhaltung vorherzusagen; und
- auf der Körperoberflächenkarte einen Abschnitt des Körpers identifiziert, der verdunkelt werden soll, und einen Abschnitt des Körpers, der während der medizinischen Bildgebungsprozedur abgebildet werden soll, durch Registrieren der Körperoberflächenkarte und der vorhergesagten Körperhaltung auf den empfangenen Bildern.

10. System (100) nach Anspruch 9, wobei die zweiten Anweisungen, wenn sie vom Prozessor ausgeführt werden, bewirken, dass der Prozessor eine Orientierung (316) einer Ultraschallbildgebungssonde (504) in Bezug auf die vorhergesagte Körperhaltung vorhersagt.

11. System (100) nach Anspruch 10, wobei die zweiten Anweisungen, wenn sie vom Prozessor ausgeführt werden, weiter bewirken, dass der Prozessor anatomische Teile identifiziert.

12. System (100) nach Anspruch 9, wobei die zweiten Anweisungen, wenn sie vom Prozessor ausgeführt werden, weiter bewirken, dass der Prozessor: eine Position einer Ultraschallbildgebungssonde (504) während eines Bildgebungsprotokolls verfolgt, die verfolgte Position der Ultraschallbildgebungssonde (504) an die Körperoberflächenkarte koppelt und die verfolgte Position in Bezug auf die Körperoberflächenkarte speichert.

13. System (100) nach Anspruch 9, wobei die zweiten Anweisungen, wenn sie vom Prozessor ausgeführt werden, weiter bewirken, dass der Prozessor: eine Position einer Ultraschallbildgebungssonde (504) während eines Bildgebungsprotokolls verfolgt und Bewegung (510) einer Ultraschallbildgebungssonde (504) basierend auf einer Vielzahl von Videobildern vorhersagt.

14. System (100) nach Anspruch 9, wobei die ersten Anweisungen, wenn sie vom Prozessor ausgeführt werden, bewirken, dass der Prozessor basierend auf Grundwahrheitsdaten den Körpertyp vorhersagt, und wobei die Grundwahrheitsdaten vorzugsweise Nicht-Bildgebungsdaten umfassen.

15. Körperliches, nichtflüchtiges, computerlesbares Medium, das ein erstes Rechenmodell, das erste Anweisungen umfasst, und ein zweites Rechenmodell, das zweite Anweisungen umfasst, speichert, die, wenn sie von einem Prozessor ausgeführt werden, bewirken, dass der Prozessor die Schritte des Verfahrens nach einem der Ansprüche 1-8 durchführt.

## Revendications

1. Procédé (600) de fourniture d'imagerie de flux de travail d'un patient pour la réalisation d'une procédure d'imagerie par ultrasons, le procédé (600) comprenant :
la réception d'images provenant d'une caméra (602) ;
la reconstruction d'une carte de surface corporelle à partir des images de la caméra (604), dans lequel la carte de surface corporelle comprend une carte de contour numérique d'une zone de surface du corps du patient ;
l'application d'un premier modèle informatique entraîné à la carte de surface corporelle pour prédire un type corporel du patient à partir d'un nombre de types corporels connus (606) ;
l'application d'un second modèle informatique entraîné aux images reçues pour prédire une pose du corps (608) sur la base du type de corps prédit ; et
l'identification, sur la carte de surface corporelle, d'une portion du corps à masquer, et d'une portion du corps à imager (610) pendant la procédure d'imagerie par ultrasons en enregistrant la carte de surface corporelle et la pose prévue du corps des images reçues.

2. Procédé (600) selon la revendication 1, comprenant en outre :
l'application du second modèle informatique entraîné pour suivre la position d'une sonde d'imagerie ultrasonore (504) au cours d'une procédure d'imagerie par ultrasons.

3. Procédé (600) selon la revendication 2, comprenant en outre l'application du second modèle informatique entraîné pour prédire une orientation (316) de la sonde d'imagerie ultrasonore (504) par rapport à la pose prédite du corps.

4. Procédé (600) selon la revendication 3, dans lequel l'application du second modèle informatique entraîné comprend en outre l'identification de parties anatomiques.

5. Procédé (600) selon la revendication 2, comprenant en outre le couplage de la position suivie de la sonde d'imagerie ultrasonore (504) à la carte de surface corporelle, et l'enregistrement de la position suivie par rapport à la carte de surface corporelle.

6. Procédé (600) selon la revendication 1, dans lequel l'application du premier modèle informatique entraîné pour prédire le type de corps comprend en outre la sélection du type de corps le plus proche de la carte de surface corporelle du patient.

7. Procédé (600) selon la revendication 2, dans lequel l'application du second modèle informatique entraîné pour suivre une position de la sonde d'imagerie ultrasonore (504) comprend en outre la prédiction du mouvement (510) de la sonde d'imagerie ultrasonore (504) sur la base d'une pluralité de trames vidéo.

8. Procédé (600) selon la revendication 6, dans lequel l'application du premier modèle informatique entraîné comprend en outre la fourniture de données de vérité terrain au premier modèle informatique entraîné pour prédire le type de corps, et dans lequel de préférence les données de vérité terrain comprennent des données non-imagerie.

9. Système (100) permettant de fournir l'imagerie de flux de travail d'un patient pour une procédure d'imagerie médicale, comprenant :
une caméra adaptée pour capturer une image tridimensionnelle (3D) (212) ;
une mémoire (130) adaptée pour stocker un premier modèle informatique comprenant des premières instructions, et un second modèle informatique comprenant des secondes instructions ; et
un processeur, dans lequel les premières instructions ou les secondes instructions, lorsqu'elles sont exécutées par le processeur, amènent le processeur à :
- recevoir des images de la caméra ;
- reconstruire une carte de surface corporelle à partir des images de la caméra, dans lequel la carte de surface corporelle comprend une carte de contour numérique d'une zone de surface du corps du patient ;
- appliquer le premier modèle informatique entraîné à la carte de surface corporelle pour prédire un type corporel du patient à partir d'un nombre de types corporels connus ;
- appliquer le second modèle informatique entraîné aux images reçues pour prédire une pose du corps sur la base du type de corps prédit ; et
- identifier, sur la carte de surface corporelle, une portion du corps à masquer, et une portion du corps à imager pendant la procédure d'imagerie médicale en enregistrant la carte de surface corporelle et la pose prévue du corps des images reçues.

10. Système (100) selon la revendication 9, dans lequel les secondes instructions, lorsqu'elles sont exécutées par le processeur, amènent le processeur à prédire une orientation (316) d'une sonde d'imagerie ultrasonore (504) par rapport à la pose prédite du corps.

11. Système (100) selon la revendication 10, dans lequel les secondes instructions, lorsqu'elles sont exécutées par le processeur, amènent en outre le processeur à identifier des parties anatomiques.

12. Système (100) selon la revendication 9, dans lequel les secondes instructions, lorsqu'elles sont exécutées par le processeur, amènent en outre le processeur à : suivre la position d'une sonde d'imagerie ultrasonore (504) pendant un protocole d'imagerie, coupler la position suivie de la sonde d'imagerie ultrasonore (504) à la carte de surface corporelle, et enregistrer la position suivie par rapport à la carte de surface corporelle.

13. Système (100) selon la revendication 9, dans lequel les secondes instructions, lorsqu'elles sont exécutées par le processeur, amènent en outre le processeur à : suivre la position d'une sonde d'imagerie ultrasonore (504) pendant un protocole d'imagerie, et prédire le mouvement (510) d'une sonde d'imagerie ultrasonore (504) sur la base d'une pluralité de trames vidéo.

14. Système (100) selon la revendication 9, dans lequel les premières instructions, lorsqu'elles sont exécutées par le processeur, amènent le processeur à prédire le type de corps sur la base de données de vérité terrain, et dans lequel de préférence les données de vérité terrain comprennent des données non-imagerie.

15. Support tangible non transitoire lisible par ordinateur qui stocke un premier modèle informatique comprenant des premières instructions et un second modèle informatique comprenant des secondes instructions, qui, lorsqu'ils sont exécutés par un processeur, amènent le processeur à effectuer les étapes du procédé selon l'une quelconque des revendications 1-8.
